# EUROPEAN PATENT APPLICATION

(11) **EP 0 908 093 A1**
(43) Date of publication of application: **14.04.1999**
(21) Application number: 96942593.3
(22) Date of filing: 20.12.1996
(51) Int. Cl.: A01K 67/027

(54) **MODEL TRANSGENIC ANIMAL FOR INTERLEUKIN-1 RELEVANT DISEASES**

(30) Priority: 22.12.1995 JP 349444/95
(71) Applicant: Hoechst Marion Roussel, Ltd., Tokyo 107-8465 (JP)
(72) Inventor: TADA, Norihiro, Urawa-shi, Saitama 336 (JP); UCHIDA, Sanae, Nippon Hoechst Marion Roussel Ltd., Kawagoe-shi, Saitama 350-11 (JP); SATOH, Masahiro, Isehara-shi, Kanagawa 259-11 (JP)
(74) Representative: Vieillefosse, Jean-Claude
(86) International application number: JP9603727
(87) International publication number: WO9723129

(57) **Abstract**

A model transgenic non-human vertebrate for interleukin-1 relevant diseases obtained by integrating into a vertebrate gene a foreign gene construction which contains a DNA sequence encoding an interleukin-1α gene in the downstream of the DNA sequence of a cytomegalovirus enhancer and the DNA sequence of chicken β-actin promoter.

## Description

### Technical Field

The present invention relates to a model transgenic non-human vertebrate for interleukin-1 relevant diseases obtained by integrating into a vertebrate genome a foreign gene construct which contains an interleukin-1α (which will hereinafter be abbreviated as "IL-1α") gene. More particularly, it relates to a model transgenic non-human vertebrate for interleukin-1 relevant diseases, said transgenic non-human vertebrate having integrated therein a foreign gene construct encoding regions of a cytomegalovirus enhancer/chicken β-actin promoter and a human IL-1α.

### Background Art

It has been elucidated (Oppenheim, J.J. et al., Immunol Today vol. 7, p. 45-56, 1986) that interleukin-1 (which will hereinafter be abbreviated as "IL-1") exhibits a variety of activities for a great number of cells and it plays an important role in not only acute inflammatory reactions but also various reactions including organic homeostasis.

Since success in cloning of cDNA of mouse IL-1 in 1984 (Lomedico, P.T. et al., Nature, vol. 312, p. 458-467, 1984), cDNAs of human, rabbit and rat IL-1 have been cloned and their structures have been disclosed. IL-1 is a simple protein without sugar chains composed of about 250 amino acids and has a molecular weight of 17.5 kD. Since IL-1 lacks a signal peptide like other secretory proteins, the mechanism of its secretion into extracellular space has not yet been elucidated sufficiently. It has been disclosed that in any one of the above-described animal species, IL-1 has two molecular types, which can be classified into type-α and type-β. It is presumed that concerning type-α, its N-terminal domain is cleaved by a calcium-dependent protease when it passes through a cellular membrane (Kobayashi Y. et al., Proc Natl Acad Sci USA, vol. 87, p. 5548-5552, 1990). The homology between IL-1 α and IL-1β in the amino acid sequences is only about 25%, while that of the same type of IL-1 between different animal species is as high as 60 to 70%. IL-1α and IL-1β are bound with the same receptor and they exhibit substantially similar bioactivities.

IL-1 is produced when a monocyte, macrophage or affinous cell thereof (Langerhans' cells, etc.) receives various stimuli from bacteria, endotoxin (LPS), complement-conjugated immunocomplex, complement factors (C3a, C5a), interferon (IFN), tumor necrosis factor (TNF), colony-stimulating factor (CSF), transforming growth factor-β (TGF-β) or virus and so on. IL-1 is also produced by keratinocyte, NK cell, T cell, B cell, blood endothelial cell, mesangium cell, synovial cell, astroglia, neutrophil or fibroblast and so on. Thus, many kinds of cells produce IL-1 and moreover, the kind of IL-1 producing cells differs depending on whether inflammation exists or not.

The recent advance in developmental engineering technology has made it possible to create transgenic animals having a foreign gene artificially integrated therein (Gordon J. and Ruddle F., Science, 214, 1244-1246, 1981). The foreign gene is introduced into an ova by a microinjection method (Gordon, et al., 1980) in which the foreign gene suctioned into a micropipet is injected into a pronucleus of the 1-cell stage ova or by infection through a virus. The ova having the foreign gene integrated therein is called a "transformed ova" (Gordon J. et al., Proc Natl Acad Sci USA, 77, 7380-7384, 1980; Jaenisch R. et al., Cell, 32, 209-216, 1983). The transformed ova can be allowed to grow by transplanting it to the genital organ or duct (oviduct or uterus) of a pseudo-pregnant recipient. The adult transgenic animal so created has the foreign gene integrated in its chromosome and can express the foreign gene under the influence of an appropriate promoter. The foreign gene so integrated is called "transgene". A transgene can be expressed in a specific to any one of the stages ranging from a fertilized ovum to an adult manner by selecting a proper promoter region and combining it with a transgene. As a result of transgene expression, the protein encoded by the transgene is produced in the transgenic animal. In particular, when the protein plays an important function for the animal, there is a possibility that a somewhat change is caused in the phenotype of the individual at some point of its development and it acquires a character analogous to some human hereditary diseases. Whether the transgenic animal has a transgene integrated in its chromosome or not can be confirmed by analysis such as PCR method or Southern blot analysis, etc. When the integration is confirmed, this animal is used for *in vivo* analysis of gene expression; for example, analysis such as Northern blot analysis or immunoassay, etc.

The gene expression is controlled by specific regions of a gene called "promoter" and "enhancer" which exist in the upstream of the gene encoding the target protein. The promoter contains a signal sequence which starts mRNA synthesis (transcription) with the DNA as a template and has a characteristic base sequence. By the action of an RNA polymerase, the synthesis of mRNA for the target protein is started. By intruducing a special DNA base sequence which is called enhancer and has a function of reinforcing the transcription efficiency of DNA into the upstream of the promoter region, the production efficiency of the target protein can be heightened.

There are some reports on the transformation of an individual by a transgene or a change of the phenotype of the individual as a result of the transformation. It is described in particular detail in the review of the following literature: Palmiter R.D. and Brinster R.L. (Annu Rev Genet, vol. 20, p. 465-499: 1986) or Gordon J.W. (In Rev of Cytobiol, vol. 115, p. 171-229: 1989). These transgenic animals can also be used in the fields of 1) analysis of gene expression on an individual level in the developmental step and 2) studies with a view to overcoming or reducing hereditary diseases.

A number of transgenic mice having various cytokine genes integrated therein have been heretofore created. For example, it is reported (Ishida Y. et al., Int Immunol 1, 113-120, 1989) that a transgenic mouse expressing human IL-2 in its thymus, spleen, bone marrow, lung, skin and other organs develops the symptoms of alopecia and pneumonia at a high ratio, but hardly shows an autoimmune-disease like pathology. In addition, it is known (Tepper R.I. et al., Cell 62, 457-467, 1990; Lewis D.B. et al., J Exp Med 173, 89-100, 1991; Muller W. et al., Eur J Immunol 22, 1179-1184, 1992; Lewis D.B. et al., Proc Natl Acad Sci USA, 90, 11618-11622, 1993) that a transgenic mouse having human IL-4 causes a change in immune response attendant on generation of T cells and abnormal regulations in an immunoglobulin isotype synthesis or osteoporosis due to reduced activities of an osteoblast and osteoclast in the osseous tissue. A transgenic mouse having mouse IL-5 is also created and over-expression of mouse IL-5 causes eosinophilia or autoantibody production (Dent L.A. et al., J Exp Med 172, 1425-1431, 1990; Tominaga A. et al., J Exp Med 173, 429-437, 1991). A transgenic mouse having human IL-6 introduced therein causes autoimmune diseases or plasmacytoma (Suematsu S. et al., Proc Natl Acad Sci USA 86, 7547-7551, 1989; Suematsu S. et al., Proc Natl Acad Sci USA 89, 232-235, 1992). In a transgenic mouse over-expressing human IL-7, human IL-8 or human GM-CSF, various lymphomas, disorder in the movement of neutrophils, cataract caused by the accumulation of macrophages in aqueous chambers and the like are recognized (Rich B.E. et al., J Exp Med 177, 305-316, 1993; Simonet W.S. et al., J Clin Invest 94, 1310-1319, 1994; Lang R.A. et al., Cell 51, 675-686, 1987). It is apparent, on the whole, that a transgenic mouse over-expressing any one of cytokines shows abnormality resulting from a change in the expression type of an immunocyte. Although there has been an attempt to create a transgenic mouse having IL-1β introduced therein, the attempt results in death and there has been no report yet that IL-1β carrying mice can survive to an adult stage (Csaikl F.F. et al., Overexpression and Knockout of Cytokines in Transgenic Mice, Jacob C.O. (ed.), 1-13, 1994).

HTLV-1px is known as a transgenic mouse which develops symptoms of arthritis (Iwakura Y. et al., Science 253, 1026-1028, 1991). Chronic arthritis which is observed in a patient suffering from adult T-cell leukemia (ATL) is reproduced in an transgenic mouse having, introduced therein, an LTR-env-px region of HTLV-1 which is a causative virus of ATL or a tax gene and such a mouse is thought to be useful as a disease model of rheumatoid arthritis. The local expression of a tax gene in joints of the HTLV-1px mouse suggests that it induces the expression of inflammatory cytokines. In fact, it has been confirmed that the expression of Il-1α, IL-1β and IL-6 are enhanced. There is also a report (Hammer R.E. et al., Cell 63, 1099-1112, 1990) on a transgenic rat which suffers from arthritis. A transgenic rat which has, introduced therein, HLA-B27 which is a major histocompatibility complex having a relationship with spondylarthritis known as a multi-organ disease and a β2 microglobulin gene develops symptoms of peripheral and myelonic arthritis, dermatitis, gastrointestinal inflammation or myocarditis, and it is thought as a model for human diseases associated with B27.

It is, however, desired to properly use plural animal models different in types in order to analyze the cause or conditions of a disease or to carry out screening of a medicament. The present inventors have carried out an extensive investigation and succeeded for the first time in the creation, as an *in vivo* IL-1 over-expression model, of a transgenic mouse which has a morbid condition closer to that of human chronic rheumatoid arthritis.

### Description of the Invention

An object of the present invention is to provide a more useful model transgenic animal for rheumatoid arthritis by making use of over-expression of IL-1 in joints and also to elucidate the function of IL-1 in the synovial membrane. More specifically, an object of the present invention is to create, based on developmental engineering technology or genetic engineering technology, a transgenic animal, preferably a transgenic mouse, which can be used as a model transgenic animal for diseases caused by over-expression of IL-1 such as autoimmune diseases, collagen diseases and osteoporosis, by over-expressing of IL-1 in an individual animal, thereby not only inducing arthritis but also exerting systemic action on it.

A transgenic mouse is created by microinjecting a gene fragment encoding human IL-1 to a pronucleus of the 1-cell stage ovum of a non-human vertebrate, preferably a mouse, transplanting the resulting ovum to a pseudo-pregnant female mouse and then breeding the mouse. The transgenic mouse so created is presumed to develop symptoms of diseases such as systemic lupus erythematosus (SLE), chronic rheumatoid arthritis or osteoporosis attendant on over-expression of IL-1. The gene fragment so injected contains a promoter which can non-specifically promote the production of IL-1 in various types of cells in the transgenic mouse. It is presumed that by the over-expression of IL-1 in an immunocyte and synovial cell under the control of such a promoter, activity (expression type) itself of these cells is changed and at the same time, an incidence ratio of arthritis is increased.

The present invention relates to a model transgenic non-human vertebrate for IL-1 relevant diseases obtained by integrating therein a foreign gene construct containing an IL-1 α gene.

The present invention relates to the model transgenic non-human vertebrate for IL-1 relevant diseases obtained by integrating therein a foreign gene construct containing an IL-1 a gene in the downstream of a DNA sequence of a cytomegalovirus enhancer and a DNA sequence of a chicken β-actin promoter. What is important in the present invention is that over-expression of IL-1 is possible in any type of tissue under the control of the powerful promoter. Described specifically, *in* *vivo* function of IL-1 covers a wide range of tissues in addition to immune-related tissues and acting mechanism of IL-1 in such tissues is almost unknown. Accordingly, there is a possibility of elucidating the action of IL-1 not only in immune-related regions but also in various tissues. With a view to elucidating its action, a chicken β-actin promoter for starting transcription in every somatic cell without limitation and a cytomegalovirus enhancer for strongly reinforcing the transcription efficiency have been integrated in a recombinant gene.

The present invention relates to the model transgenic non-human vertebrate for IL-1 relevant diseases, wherein said IL-1α gene is derived from a human being.

The present invention relates to the model transgenic non-human vertebrate for IL-1 relevant diseases, wherein the model transgenic non-human vertebrate is a mouse.

The present invention relates to the model transgenic non-human vertebrate for IL-1 relevant diseases, wherein the IL-1 relevant disease is a chronic inflammatory disease.

The present invention relates to the model transgenic non-human vertebrate for IL-1 relevant diseases, wherein the IL-1 relevant disease is chronic rheumatoid arthritis.

The present invention relates to the model transgenic non-human vertebrate for IL-1 relevant diseases, wherein the IL-1 relevant disease is systemic lupus erythematosus.

The present invention relates to the model transgenic non-human vertebrate for IL-1 relevant diseases, wherein the IL-1 relevant disease is osteoporosis.

The model transgenic non-human vertebrate for IL-1 relevant diseases according to the present invention characterizes in that it shows articular swelling caused by immune response exasperation in the joints of limbs, infiltration of lymphocytes into joints, proliferation of synovial cells, fracture of a bone or cartilage, autoantibody production, damage of blood endothelial cells or osteoporosis. It has been disclosed that the transgenic animal according to the present invention exhibits chronic-rheumatoid-arthritis-like morbid conditions owing to over-expression of IL-1. Accordingly, the present invention provides a line useful for *in vivo* study of the mechanism of chronic rheumatoid arthritis caused by the local over-expression of IL-1 in joints. In osteoporosis, osteogenesis exceeding bone resorption leads to a decrease of osseous tissues, resulting in symptoms such as dorsolumbar pain and fracture. Judging from the facts that an amount of IL-1 produced by patients suffering from osteoporosis is large and IL-1 has a strong bone resorption accelerating action, a change in bone due to over-expression of IL-1 is expected. SLE is, on the other hand, a disease showing an abnormality in an immune control mechanism. In this disease, reinforcement of autoantibody production due to functional exasperation of B cells is recognized and owing to an immunocomplex so formed, complement-dependent histionic disorders occur. It is known that as one of the causes for such immune abnormalities, there exist abnormalities in the production or reactivity of a cytokine such as IL-1.

The participation of IL-1 is also suggested as a cause for the Lyme disease. The Lyme disease is carried by the acarid and its pathogen is a spirochete. The disease starts with a characteristic dermal lesion, which advances into chronic arthritis. It has been revealed (Habicht, G.S. et al., J. Immunol., 134, 3147, 1985) that IL-1 is induced from a monocyte by a spirochete.

As described above, over-expression of IL-1 is found almost all over the body so that the transgenic mouse of the present invention can be used for elucidation of the onset mechanism of the diseases which are presumed to result from the over-expression of IL-1 or for research of the remedies of such diseases. For example, a medicament to be researched is administered to a control animal, that is, an animal group (non-transgenic mouse, etc.) which is not the transgenic mouse of the present invention and the transgenic mouse of the present invention at the same time. This medicament will be administered continuously for a period exceeding the period sufficient for bringing about an improvement in the morbid condition of the transgenic mouse. After administration of the medicament for over the sufficient period, the transgenic mouse and non-transgenic mouse used as a control are subjected to analysis of articular tissues or the like. The efficiency of the medicament can be determined by comparing the above-described parameter between them.

The present invention relates to the model transgenic mouse for IL-1 relevant diseases, wherein the mouse is βAIL-1 α-1705 or βAIL-1α-1706.

The present inventors created the transgenic mouse which suffers from IL-1 relevant diseases by conducting systemic over-expression of a IL-1α gene. In the mouse so created, observed are production of an autoantibody, proliferation of neutrophils, arthritis, activity exasperation of immunocytes and the like. Such morbid states resemble to those of the above-described diseases. The transgenic mouse of the present invention therefore provides a line for elucidation of the onset mechanism of various IL-1 relevant diseases resulting from over-expression of IL-1 and *in vivo* screening of remedies for these diseases.

The line of the transgenic animal according to the present invention is maintained by *in vitro* fertilization. A transgenic animal coming into existence can be bred in a similar manner to that employed generally for animals and no particular breeding or feed are required.

An object of the present invention is to provide a transgenic animal having a DNA sequence necessary for strongly causing production of IL-1 in an articular tissue or other tissues, in other words, having a recombinant DNA. The present invention exhibits its usefulness when the transgenic animal is used for elucidation of *in vivo* action of IL-1, elucidation of the onset mechanism of the above-described IL-1 relevant diseases and *in vivo* screening of the remedies for these associated diseases.

### Brief Description of the Drawings

FIG. 1 is a a plasmid map of plasmid pβA/hIL-1α-1 having DNA sequence encoding IL-1α gene in the downstream of the DNA sequence of a cytomegalovirus enhancer and the DNA sequence of a chicken β-actin promoter and a rabbit β-globin gene.
FIG. 2 is a microphotograph of samples of the calvaria of βAIL-1α-1705 FO mice (A) and non-transgenic mice (B), all being 4 weeks old, stained according to a hematoxylin-eosin staining.
FIG. 3 is a microphotograph of samples of the femurs of βAIL-1α-1705 FO mice (A) and non-transgenic mice (B), all being 4 weeks old, stained according to a hematoxylin-eosin stain.
FIG. 4 is a microphotograph of samples of the spleen cells of βAIL-1α-1705 FO mice (A) and non-transgenic mice (B), all being 4 weeks old, stained according to a hematoxylin-eosin stain.

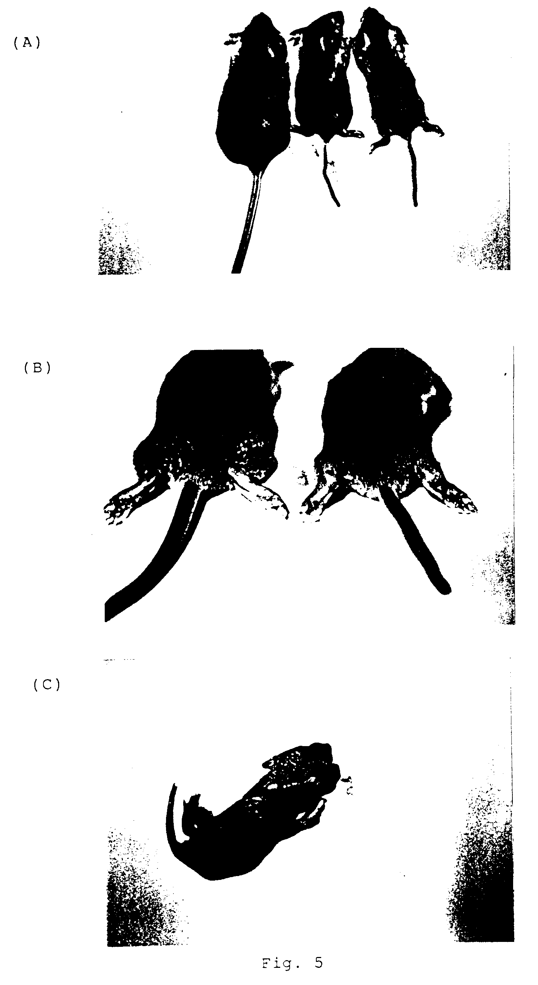

FIG. 5 (A) is a whole body photograph of the morphological pictures of a βAIL-1α-1705 transgenic mouse (at the right end), a βAIL-1α-1706 transgenic mouse (at the center) and a non-transgenic littermate (at the left end), all being 5 weeks old, and (B) is a photograph of the morphological pictures of the hind legs with swollen periarticular parts of a βAIL-1α-1705 transgenic mouse (at the right) and of the hind legs of a non-transgenic mouse (at the left), while (C) is a whole-body photograph of the morphological pictures of a 9-day-old F1 transgenic mouse (βAIL-1α-1705 -2) created according to *in vitro* fertilization.

### Description of the Preferred Embodiments

The present invention will hereinafter be described more concretely by examples.

An object of the below-described examples is to completely disclose and describe the preparation processes of a gene fragment, recombinant gene construct, transgenic mouse and the like. It should, however, be borne in mind that the present invention is not limited to or by these examples.

### Example I: Construct of Plasmid pβA/hIL-1α-1

The target gene to be expressed in a mouse was prepared as follows:

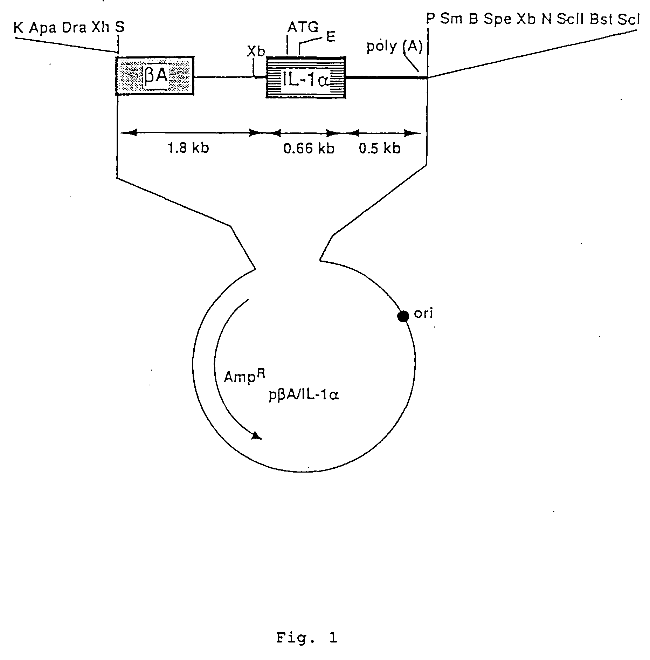

First, a vector for expressing the above target gene was prepared. From pCAGGS (Niwa H. et al., Gene, vol. 108, p. 193-200, 1991) which was an expression vector of a mammalian having a chicken β-actin promoter and a cytomegalovirus in the upstream of the promoter, a 2.3 kb fragment was cleaved by the digestion with SalI/PstI. The fragment was then inserted into the SalI/PstI site of pBluescript (purchased from Stratagene Corp.) which was a cloning vector, whereby a pBsCAG-2 vector was constructed. The cytomegalovirus enhancer/chicken β-actin promoter has been recognized to have strong activity in various types of cells in mammals. At present, it is proved to be useful as a promoter system capable of strongly promoting the *in vivo* expression of a foreign gene. The 2.3 kb fragment contained, in addition to the abovedescribed enhancer/promoter, a part (containing the second intron, third exon and 3'- non-translated region) of a rabbit β-globin gene. In general; the target gene to be expressed, such as cDNA, is inserted into the EcoRI site of the third exon of the rabbit β-globin gene. It is known that the *in vivo* transcription efficiency of the target gene is improved by the existence of intron (Brinster R.L. et al., Proc. Natl. Acad. Sci. USA, vol. 85, p. 836-840, 1988). Into the EcoRI site of that pBsCAG-2, the full length (660 bp) of the cDNA of human IL-1α [obtained from hIL-1αH2H3 plasmid (March et al.,Nature, 315, p. 641, 1985)], which was the target gene, was inserted, whereby plasmid pβA/hIL-1α-1 (5.96 kb) for the expression in a transgenic mouse was constructed (FIG. 1). For the introduction of DNA into the 1-cell stage ovum of a mouse, a gene was isolated from the recombinant gene construct by the digestion with ScaI/SalI/BamHI and then provided for use.

The construction was subjected to various operations such as digestion and ligation of DNA and isolation of a DNA fragment, for which standard DNA recombinant technology of Maniatis T. et al. (Molecular Cloning, A laboratory Manual, 1982) was employed. The DNA sequence at the periphery of the inserted portion was confirmed by the DNA sequencing analysis.

### Example 2: Injection of a foreign gene (βA-hIL-1α) to a fertilized ovum of a mouse, transplantation of the resulting ovum and confirmation of the transgene so introduced.

As experimental animals, male C57BL/6N mice and female B6C3F1 mice (C57BL/6N x C3H/HeN) were used for collection of fertilized ova and female ICR mice were used as a recipient to be transplanted with the fertilized ovum after DNA injection. To induce pseudo-pregnancy, the recipients to be transplanted were crossed with male vasectomized ICR mice. First, in order to induce super ovulation, pregnant mare's serum gonadotropin (PMSG) and human chorionic gonadotropin (hCG) were peritoneally administered to the female B6C3F1 mice, each in an amount of 5 IU, at intervals of 48 hours. Just after the administration, the female mice so treated were put into the same cages with the male C57BL/6N mice, respectively. Next day, female B6C3F1 mice having a plug in their vagina were judged as those who had succeeded in mating. The mice whose mating was confirmed were sacrificed and the fertilized ovum (the pronucleate stage ovum) surrounded by the cells of a granular layer and cumulus oophorus was collected from the ampulla of the oviduct of the respective mice. The fertilized ovum so collected was introduced into a M16 culture medium (Whittingham D.G. J. Reprod. Fert., Suppl. vol. 14, p. 7-21, 1971) containing 1% hyaluronidase (Mochida Pharmaceutical Co., Ltd.), whereby the cells of the granular layer and cumulus oophorus were removed. The incubation was performed in the M16 culture medium in a gaseous phase of 5% CO₂ and 95% air at 37° C until the injection of DNA. The incubation was carried out by allowing the fertilized ovum to float in microdrops of 50 µl of the M16 culture medium added dropwise in a suspension culture dish (No. 171099, Nunc Corp.) of 35 mm in diameter, with the upper part being covered with liquid paraffin (White light mineral oil, Fisher Corp.).

The foreign gene was extracted after amplification of the plasmid pβA/hIL-1α-1, which had been prepared above, with a host *Escherichia coli*. The plasmid was purified further by ultracentrifugation with cesium chloride, removal of ethidium bromide and dialysis. The plasmid so purified was digested with restriction enzymes SalI, BamHI and ScaI, followed by isolation of the target foreign gene (βA-hIL-1α, 3.0 kb) by electrophoresis on 0.8% agarose gels. The foreign gene diluted with a phosphate buffer (pH 7.2) just before the injecting operation was employed. The foreign gene was injected into the fertilized ovum in accordance with the previous report (Hogan B. et al., In Manipulating the Mouse Embryo., Cold Spring Harbor Laboratory Press, 1986). Described specifically, the fertilized ovum was retained by a holding glass pipette in a culture medium and then about 2 pl (2,000 copies) portions of DNA solution were injected into the male pronucleus of the fertilized ovum by using a minute glass pipette for injection.

After injection, the resulting fertilized ovum was transplanted into the oviduct of a mature female ICR mouse on Day 1 of pseudo-pregnancy, said female mouse having been mated with a male mature vasectomized ICR mouse. The mouse was kept until the full term and a baby mouse was created. After parturition, the baby mouse so created was weaned from the breast when it was four weeks old and for identification of an individual mouse, the tip of its tail was cut by about 1 cm under anesthesia and its ear was punched. From the tissue of the tail, chromosomal DNA was extracted, followed by purification. It was confirmed by the Southern blot analysis that the transgene fragment so introduced had been integrated in the chromosome of the mouse. Described specifically, 10 µg of the chromosome DNA completely digested with restriction enzymes EcoRI and BamHI were subjected to electrophoresis on 0.8% agarose gels, followed by transfer of the chromosomal DNA to a nylon filter (GeneScreenPlus, NEN, USA). The filter was air-dried and was then subjected to hybridization. Hybridization was carried out with the hIL-1α gene fragment as a probe in a manner known per se in the art (Maniatis T. et al., 1982) and integration of the transgene fragment into the mouse chromosome was confirmed.

The results are shown below in Table 1. In the experiments made twice in total, the gene was injected into 133 fertilized ova and of these, 86 fertilized ova (65%) were alive after the injecting operation. Those living ova were all transplanted to four recipients and then, three recipients became pregnant. By the Cesarean section on Day 19 after pregnancy, 24 baby mice (18%) were taken out. After resuscitation, the baby mice were left in the care of foster parents (female ICR mice) which had been prepared and controlled in advance to finish the delivery on the same day. The transgene was found in the chromosome DNA extracted from 11 baby mice (8%), out of 24 baby mice. Two of them were, however, killed by the foster parent so that they could not be provided for subsequent analysis.

**Table 1**

| Frequency of experiment | The number of ova injected :A | The number of ova transplanted :B (succeeding ratio of injection: B/A) | The number of survival baby mice: C (survival ratio: C/A) | Transgenic mice: D (gene introduction ratio:D/A) |
|---|---|---|---|---|
| 2 | 133 | 86 (65%) | 24 (18%) | 11 (8%) |

The nine transgenic mice which had remained without being killed by foster parent mice grew up until weaning. From those nine transgenic mice, F1 mice were created, respectively. As a result of studying the transmission of transgene, all of the nine transgenic mice transmitted their transgene to F1 mice. The characteristics of the transgenic mice so created are shown in Table 2.

**Table 2**

| Line No. | Sex | Transmission of a transgene to F1 | Expression in each tissue | Finding |
|---|---|---|---|---|
| βAIL-1α -1402 | Female | + | - | Normal |
| βAIL-1α -1604 | Female | + | - | Normal |
| βAIL-1α -1605 | Female | + | - | Normal |
| βAIL-1α -1609 | Male | + | - | Normal |
| βAIL-1α -1701 | Male | + | - | Normal |
| βAIL-1α -1705 | Male | + | + | Small size, articular swelling, etc. |
| βAIL-1α -1706 | Male | + | + | Small size, articular swelling, etc. |
| βAIL-1α -1707 | Female | + | - | Normal |
| βAIL-1α -1708 | Female | + | - | Normal |

The transgenic mice so created transmitted their transgene to their progeny (F1) in all the lines, but expression in each tissue was recognized in only two lines (βAIL-1α-1705 and βAIL-1α-1706).

Those transgenic mice of the first generation in which the transgene has been expressed each reached weaning (4 weeks old) but its size was very small (about half of the weight of a non-transgenic mouse). At the time of weaning, a marked swelling had already been observed around the joint region of the four legs, which became worse with aging. The observation was continued while the mouse was kept until it became 14 weeks old. Closing of the eyelid (due to the gumming with the eye mucus around the eye) and epilation were confirmed when the mice became 7 weeks old and 13 weeks old, respectively. The lower portions of the hind begs with articular swelling were bent inside and thus started deformation when the mice became 10 weeks old. At that time, it was observed that owing to the thorough paralysis of the hind legs, the mouse moved using only the forelegs.

The present inventors judged that it was impossible to create a progeny (F1) by the natural mating of such a transgenic mouse and tried to obtain F1 by the *in vitro* fertilization. The *in vitro* fertilization was carried out in a manner known per se in the art (Toyoda Y., Yokohama M., Hoshi T., Japan J. Anim. Reprod., 16, 147-151, 1971). Descried specifically, cauda epididymidis was excised from two lines of the transgenic mice (F0) which had been sacrificed by the direct exsanguination from its heart and was introduced into 400 µl of a TYH culture medium, which had been prepared in advance, in a suspension culture dish (Nunc Corp.) (said TYH culture medium is prepared by successively adding, to 100 ml of extra-pure water, 0.6976 g of NaCl, 0.0356 g of KCl, 0.0251 g of CaCl₂ ·2H₂O, 0.0162 g of KH₂PO₄, 0.0293 g of MgSO₄ ·7H₂O, 0.2106 g of NaHCO₃, 0.011 g of sodium pyruvate, 0.1 g of glucose, 0.4 g of bovine serum albumin, 0.0075 g of penicillin G, 0.005 g of streptomycin and 0.0002 g of phenol red to dissolve the later in the former. The resulting solution is sterilized by passing it through a Millipore filter and then provided for experiment. Reference cited: Toyoda, Y., Yokohama, M. and Hoshi, T., Jpn. J. Anim. Reprod., 16, 147-151, 1971). The cauda epididymidis so introduced was finely cut by ophthalmologic scissors, whereby sperms were collected. The transgenic-mouse-derived sperms were incubated for one hour and then added as a 10 µl sperm suspended solution to 400 µl of a TYH culture medium containing unfertilized ova collected in advance from female B6C3F1 mice. Incubation was performed under that condition at 37° C for 24 hours in a gaseous phase of 5% CO₂ and 95% air. The ova entering into the 2-cell stage after 24-hour incubation were judged as fertilized ova and some portions of them were transplanted into the oviduct of the ICR recipient mouse on day 1 of pseudo-pregnancy. The other portions were frozen and stored in accordance with the previous report (Tada N. et al., Theriogenology, 40, 333-344, 1993). The number of fertilized ova and transplantation results are shown below in Table 3.

**Table 3**

| Line No. | The number of fertilized ova | The number of transplanted ova | The number of transplanted recipients | The number of pregnant recipients | The number of baby mice born (%) |
|---|---|---|---|---|---|
| βAIL-1α -1705 | 444 | 131 | 8 | 7 | 52 (40) |
| βAIL-1α -1706 | 384 | 98 | 5 | 5 | 27 (28) |

The baby mice created by *in vitro* fertilization and transplantation included transgenic mice (F1) of each line. The F1 transgenic mice of βAIL-1α-1705 each showed a similar phenotype to F0 and from them; slow growth and arthritis were observed in both F0 and F1 mice. Compared with the phenotype of F0, their abnormal degree was eminent. The F1 transgenic mice of βAIL-1α-1706, on the other hand, were not so abnormal as F0. It was found that in the case of F1, the onset of arthritis tended to appear more slowly.

### Example 3: Expression of transgene-derived mRNA

The expression of transgene-derived mRNA was confirmed in all of the nine IL-1α transgenic line F0 mice by the Northern blot analysis. First, the total RNA was isolated from the brain, liver, kidney, small intestine, testis, skeletal muscle, skin and tibia of the transgenic F0 mice and their non-transgenic littermates. The Northern blot analysis was conducted by subjecting 20 µg of the total RNA to electrophoresis through 1.1% agarose/1.1M formaldehyde gels and then transferring it to a nylon-membrane filter. Prehybridization was carried out at 42° C for 2 hours in a hybridization solution [containing 5 x SSC (1 x SSC = 0.15 M NaCl, 15 mM Na-citrate, pH7.4), 50% formamide, 5 mM EDTA, 5 mg/ml of denatured salmon DNA, 5 x Denhardt's solution and the like]. Then cDNA probes (human IL-1α gene fragment) primed randomly were denatured by boiling, followed by the addition to the hybridization solution, whereby hybridization was performed. The reaction was carried out at 42° C for 18 hours and rinsing was conducted in 0.1 x SSC/0.1% SDS at 56° C for 20 minutes. The filter was exposed at -80° C for 24 to 72 hours (by using intensifying screen + Kodak XAR-5 film). The above-described main organs of F0 transgenic mice of 9 lines were subjected to Northern blot analysis. In all the lines except for βAIL-1α-1705 and βAIL-1α-1706 lines, no expression of IL-1α was recognized. In the kidney, small intestine, skin and tibia of the βAIL-1α-1705 line, strong expression of IL-1α was recognized, while in the brain, liver and skeletal muscle, the expression was weak. In addition, its expression was hardly recognized in the testis. Although the βAIL-1α-1706 line on the whole showed a weak expression tendency compared with the βAIL-1α-1705 line, strong expression was recognized only in the skeletal muscle. In the brain, liver, kidney, small intestine, skin and tibia, comparatively weak expression was observed but almost no expression was found in the testis. Incidentally, βAIL-1α -1705 and βAIL-1α-1706 were analyzed but results and diagram of these two lines shown below exhibit almost similar morbid conditions so that a description will next be made based on only the finding of βAIL-1α-1705 F0 mice.

### Example 4: Pathological analysis of each of the organs of a transgenic mouse

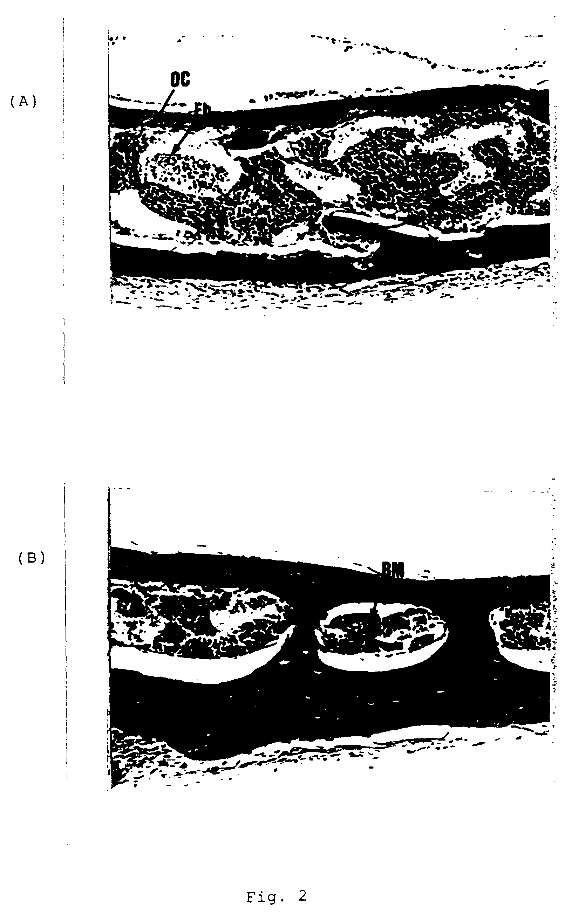

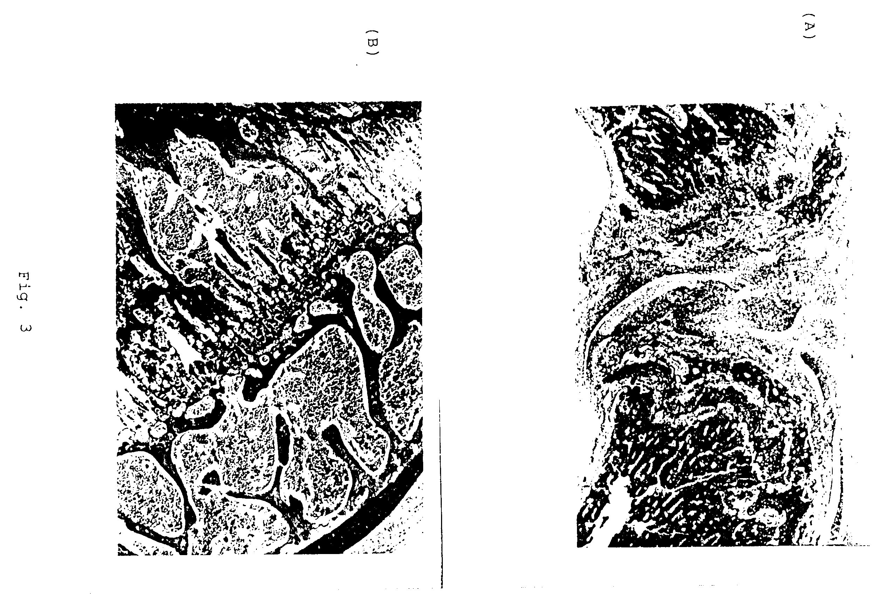

After deep anesthesia with sodium pentbarbiturate, four-week-old βAIL-1α-1705 F0 mice were sacrificed by the exsanguination from their hearts. Then, the brain, liver, kidney, thymus, spleen, pancreas, lung, heart, testis, small intestine, large intestine, calvaria, skin, femur (knee joint) and eyeball were excised from the mice. For each of the above-described organs so excised, a continuous thin-sliced sample of about 2 mm was prepared by fixing its tissue in a 10% formalin buffer and then embedding it in paraffin. The osseous tissue was subjected to decalcifying treatment by immersion in 14% EDTA for about one week prior to the preparation of a thin-sliced sample. The samples so obtained were subjected to hematoxylin and eosin stain. Subsequent to staining, the samples were observed for abnormality under a light microscope and microphotographs of some of the samples were taken (FIGS. 2 and 3).

The analytical results of the samples stained with hematoxylin and eosin will be shown below. In the pathological sections of the thymus, shrinkage of the cortex was found in the transgenic mice compared with the non-transgenic mice.

In the pathological sections of the spleen, an increase in the neutrophils, exasperation of extramedullary hemopoiesis and a slight increase in plasmacyte-like cells were found in the case of the transgenic mice, while no such changes were found in the spleen of the non-transgenic mice.

Concerning the pathological sections of the lung, the lung of the transgenic mice had a thick alveolar wall owing to insufficient alveolar dilation and infiltration of neophils were found, while no abnormalities were found in the lung of the non-transgenic mice.

In the pathological sections of the kidney, spreading of the glomerular mesangium region and a sclerogenic change under the hypertrophic condition were found in the transgenic mice, while no change was recognized in that of the non-transgenic mice.

In the pathological sections of the calvaria, an eminent bone resorption image due to an increase in the number of osteoclasts (the arrow OC), an increase in the number of neutrophils in the medullary space (the arrow BM) and substitution of the bone marrow by the fibrous tissue (the arrow Fb) were found in the transgenic mice, while no such changes were recognized in the calvaria (FIG. 2B) of the non-transgenic mice.

In the pathological sections of the skin, shrinkage of the dermal tissue was found to a slight extent in the transgenic mice, while no such change was found in the skin of the non-transgenic mice.

In the pathological sections of the femur, a marked increase in the number of the myeloic cells (the allow BMC), the bone trabecula resorption image (the arrow Rs) of the osteoclast and the substitution of the bone mallow by the fibrous tissue (the arrow Fb) due to osseocartilaginous fracture were observed in the transgenic mice. In addition, infiltration of neutrophils to famous joints and an increase in the number of synovial cells and pannus formation of joints to a slight extent were recognized. No such changes were, on the other hand, found in the femur of the non-transgenic mice (FIG. 3B).

In the pathological sections of the eyeball, tylosis of the anterior epithelium of cornea was recognized in the transgenic mice, which is presumed to result from an increase in the number of stratified squamous epithelium cells. Infiltration of granulocytic cells was not observed. No such changes were, on the other hand, found in the non-transgenic mice.

Incidentally, in the organs other than those judged to have abnormalities, that is, brain, liver, pancreas, heart, testis, small intestine and large intestine, no abnormalities were found as a result of pathohistological research.

As described above, IL-1 exhibits not only the action as an endogenous pyrogen factor, leukocytosis-promoting factor, acute phase protein-promoting factor, lymphocyte activating factor or B-cell activating factor but also various activities for a great number of cells. It has been revealed that IL-1 plays an important role in various reactions including homeostasis of a living body as well as in acute inflammation. Particularly, the relationship between IL-1 and chronic inflammation type diseases has drawn attentions. As a pathohistological change typical to the mice of the present invention, an image of granulomatous proliferative inflammation showing inflammatory changes in the famous joint parts can be given. Recognized were an image of the articular cartilage or osseous tissue destroyed by the fibrous tissue, partial pannus formation or increase in the synovial cells, which were changes regarded as arthritis. Since changes such as a resorption image of the bone by osteoclasts or substitution of the bone mallow by the fibrous tissue, IL-1 was presumed to have a direct influence on the bone in addition to the joints. Myeloic cell proliferation in the bone mallow or spleen and infiltration of neutrophils in the lung is presumed to be a secondary change cause by arthritis, but there is a report (Larsen C.G. et al, Science, 243, 1464-1466, 1989) that adhesion of such cells is heightened by the acceleration of chemotaxis of lymphocytes or neutrophils by IL-8 whose production is induced by Il-1, which suggests a direct influence of over-expression of IL-1.

### Example 5: Confirmation of cytokine-producing capacity of transgenic-mouse-derived spleen cells stimulated by mitogen

Using spleen cells of the transgenic mice, a change in the phenotype of immunocytes due to over-expression of IL-1 was observed.

The spleen excised from the transgenic mouse was homogenized by a glass-made homogenizer. Then, erythrocytes were subjected to hemolysis in a Tris buffer (0.75% NH₄Cl, 5.6mM Tris-HCl, pH 7.65), followed by the removal of the remaining destroyed erythrocytes through a cell strainer (Falcon 2350). The spleen cells so isolated were adjusted with 10%-FCS-containing RPMI 1640 to give a concentration of 5 x 10⁶/ml, which was designated as a spleen cell suspension. Concanavalin A (Conc A; E.Y. Labs, Inc.) and lipopolysaccharide (LPS; Difco Laboratories) were added to the spleen cell suspensions to give a concentration of 2 µg/ml and 5 µg/ml, respectively. Anti-mouse CD3 antibody (B.M.Y.) was diluted with PBS to give a concentration of 5 µg/ml and it was added to the 24-well incubation plate in an amount of 250 µl per well. After incubation at room temperature for 2 hours, the wells were rinsed with 2%-FCS-containing RPMI 1640. In the above operations, the spleen cell suspension was added to each of the 24 wells in an amount of 1 ml. After added, the cell suspension incubated in an incubator of 5% CO₂ at 37° C for 2 days was centrifuged at 8,000 rpm for 10 minutes and its supernatant was collected. The determination of the amounts of cytokines (Mouse IL-2, Mouse IL-4, Mouse IL-6 and INF-γ: Biosource International, Mouse IL-1α and Mouse TNFα: Genzyme, Human IL-1α: R & D Systems Inc.) contained in the supernatant of the spleen cell suspension was carried out using a commercially available ELISA kit at an optical density (OD) of 450 nm in accordance with the procedure of its protocol.

Results of the cytokine-producing capacity of the transgenic-mouse-derived spleen cells caused by the mitogen stimulation are shown in Table 4.

**Table 4**

| | | Cytokine | | | | | | |
|---|---|---|---|---|---|---|---|---|
| Animal | Mitogen | IL-4 | IL-2 | IFNγ | IL-6 | TNFα | m-IL-1 α | h-IL-1 α |
| Tg | non | 0.126 | 0.07 | 0.057 | 0.101 | 0.087 | 0.09 | 0.679 |
| (βAIL-1 α-1705) | ConA | 0.142 | 2.2 | 1.102 | 1.201 | 0.497 | 0.524 | 1.414 |
| | CD3 | 0.369 | 0.272 | 1.797 | 2.113 | 0.805 | 0.881 | 1.846 |
| | LPS | 0.122 | 0.052 | 0.055 | 2.2 | 0.968 | 0.464 | 1.093 |
| | Collagen - 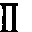 | 0.119 | 0.057 | 0.064 | 0.831 | 1.231 | 0.287 | 0.989 |
| non-Tg | non | 0.153 | 0.091 | 0.052 | 0.098 | 0.097 | 0.114 | n.d. |
| | ConA | 0.176 | 0.109 | 1.56 | 0.755 | 0.793 | 0.346 | n.d. |
| | CD3 | 0.377 | 0.081 | 2.04 | 0.935 | 0.994 | 0.255 | n.d. |
| | LPS | 0.132 | 0.078 | 0.057 | 0.764 | 0.329 | 0.397 | n.d. |
| | Collagen - 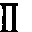 | 0.111 | 0.08 | 0.064 | 0.205 | 0.333 | 0.392 | n.d. |
| DBA1:CFA | non | 0.12 | 0.109 | 0.068 | 0.095 | 0.163 | 0.112 | n.d. |
| | ConA | 0.131 | 0.46 | 1.516 | 0.485 | 0.965 | 0.267 | n.d. |
| | CD3 | 0.244 | 1.176 | 1.787 | 0.652 | 1.346 | 0.27 | n.d. |
| | LPS | 0.112 | 0.042 | 0.055 | 0.803 | 0.392 | 0.473 | n.d. |
| | Collagen - 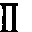 | 0.115 | 0.089 | 0.071 | 0.283 | 0.437 | 0.435 | n.d. |
| DBA1:RA | non | 0.117 | 0.135 | 0.08 | 0.196 | 0.311 | 0.16 | n.d. |
| | ConA | 0.124 | 0.947 | 1.416 | 0.608 | 0.922 | 0.223 | n.d. |
| | CD3 | 0.227 | 1.273 | 1.686 | 0.55 | 1.012 | 0.297 | n.d. |
| | LPS | 0.118 | 0.04 | 0.095 | 1.417 | 0.765 | 0.54 | n.d. |
| | Collagen - 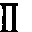 | 0.097 | 0.073 | 0.168 | 0.466 | 0.823 | 0.59 | n.d. |
| n.d. = not detected | | | | | | | | |

The term "mitogen" (division promoting factor) means a substance which activates lymphocytes and induces and promotes cell division. Representative examples include lectins derived from plants such as PHA (phytohemagglutinin A) and Con A and bacteria-derived LPS. It is known that mitogens such as PHA and Con A stimulate T cells, while LPS and the like stimulate B cells.

The transgenic-mouse-derived spleen cells produce human IL-1α spontaneously even without the stimulation of a mitogen. It has been found on the whole that the stimulation of various mitogens tend to exasperate production of cytokines except IL-4 in the transgenic-mouse-derived spleen cells compared with the non-transgenic-mouse-derived ones. For example, the stimulation of the transgenic-mouse-derived spleen cells by Con A (concanavalin A) exasperated the production of IL-2, IFN γ, IL-6 and human IL-1α, with the exasperation of IL-2 being particularly eminent. Such an exasperation pattern of cytokine production is clearly different from that of DBA/1 mouse (DBA1 : CFA) to which only an adjuvant has been administered or that of DBA/1 mouse (DBA1 : RA) which has arthritis induced by the administration of type-II collagen. This suggests that external stimuli such as infection and antigen stimulation rapidly exasperate cytokine production, thereby exasperating immune response (immunocompetence) at the same time. It has been confirmed that human IL-1α also exists in the blood of the transgenic mouse. The existence of human IL-1α has not been confirmed from the supernatant of the incubated non-transgenic-mouse-derived spleen cells and the blood of the mouse.

### Example 6: Detection of antinuclear antibody from transgenic-mouse-derived spleen cells

As described above, it is presumed that excessive immune response occurs in the transgenic mouse of the present invention owing to the over-expression of human IL-1α. In order to confirm the possibility of the autoimmune diseases, an attempt to detect an antinuclear antibody from the transgenic-mouse-derived spleen cells was made. The term "antinuclear antibody" means an antibody which is detectable in the blood of a patient suffering from an autoimmune disease and whose antigen is a cell nuclear component. It can be detected from the blood of an SLE patient at high frequency and can also be detected from the blood of a patient suffering from rheumatoid arthritis, scleroderma or thymoma. Chromatin components such as single-stranded DNA, double-stranded DNA, histone and poly-ADP ribose and soluble components in the necleoplasm such as RNA protein act as an antigen. The antibody reactive with DNA is used for the diagnosis of SLE.

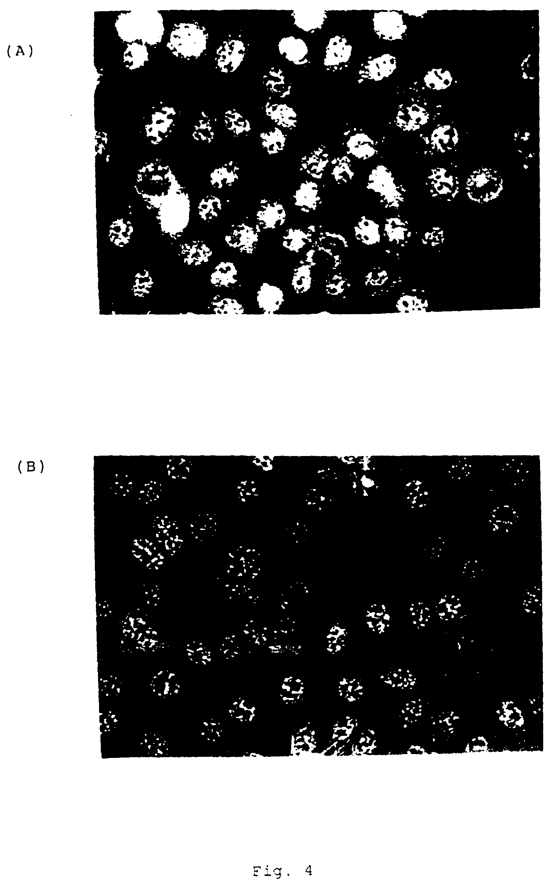

A microphotograph of the transgenic-mouse-derived spleen cells having an antinuclear antibody stained with FITC stain is shown in FIG. 4. The whole cytoplasm of the spleen cells (FIG. 4A) of the transgenic mouse is stained clearly, from which the existence of an antinuclear antibody can be confirmed. The spleen cells (FIG. 4B) of the non-transgenic mouse is stained a little but its stained property is apparently weak compared with that of the transgenic-mouse-derived spleen cells. The stained property of the non-transgenic-mouse-derived spleen cells is presumed to be nonspecific.

A whole body photography of the transgenic mouse is shown in FIG. 5. FIG. 5A are whole body photographs of a 5-week-old βAIL-1α-1705 transgenic mouse (at the right end), βAIL-1 α-1706 transgenic mouse (at the center) and non-transgenic mouse (at the left end) of the same litter. Compared with the non-transgenic mouse, two transgenic mice are apparently small and weigh about half of the non-transgenic mouse. From the photographs, it can be confirmed that these two transgenic mice have curved tails. In addition, forelegs of the 5-week-old transgenic mouse (βAIL-1α-1705) are found to have periarticular swelling compared with those of the non-transgenic mouse.

A photograph of the transgenic mouse (βAIL-1α-1705) having hind legs with periarticular swelling is shown in FIG. 5B. The hind legs (at the right) of the transgenic mouse show apparent swelling compared with those (at the left) of the non-transgenic mouse. A whole body photograph of a 9-day-old F1 transgenic mouse (βAIL-1α-1705-2) created by *in vitro* fertilization is shown in FIG. 5C. It is found that the mouse so created has already had marked periarticular swelling at its four legs and besides, is slow in growth.

The transgenic animal according to the present invention exasperates activities of immunnocytes, intra-articular synovial cells and the like owing to the systemic overexpression of human IL-1α, thereby developing symptoms of chronic rheumatoid arthritis. Its morbid state is remarkably similar to that of chronic rheumatoid arthritis including collagenosis in human beings so that the transgenic animal becomes a useful model of such diseases and is therefore valuable in the development of pharmaceuticals.

## Claims

1. A model transgenic non-human vertebrate for interleukin-1 relevant diseases obtained by integrating therein a foreign gene construct which comprises an interleukin-1α gene.

2. The model transgenic non-human vertebrate for interleukin-1 relevant diseases according to claim 1, wherein said foreign gene construction comprises a DNA sequence encoding an interleukin-1α gene in the downstream of a DNA sequence of a cytomegalovirus enhancer and a DNA sequence of a chicken β-actin promoter.

3. The model transgenic non-human vertebrate for interleukin-1 relevant diseases according to claim 1 or 2, wherein said interleukin-1α gene is derived from a human being.

4. The model transgenic non-human vertebrate for interleukin-1 relevant diseases according to any one of claims 1 to 3, wherein said model transgenic non-human vertebrate is a mouse.

5. The model transgenic non-human vertebrate for interleukin-1 relevant diseases according to any one of claims 1 to 4, wherein the interleukin-1 relevant disease is a chronic inflammatory disease.

6. The model transgenic non-human vertebrate for interleukin-1 relevant diseases according to any one of claims 1 to 4, wherein the interleukin-1 relevant disease is chronic rheumatoid arthritis.

7. The model transgenic non-human vertebrate for interleukin-1 relevant diseases according to any one of claims 1 to 4, wherein the interleukin-1 relevant disease is systemic lupus erythematosus.

8. The model transgenic non-human vertebrate for interleukin-1 relevant diseases according to any one of claims 1 to 4, wherein the interleukin-1 relevant disease is osteoporosis.

9. The model transgenic mouse for interleukin-1 relevant diseases according to any one of claims 4 to 8, which is β AIL-1-α1705 or βAIL-1-α1706 mice.
